# EUROPEAN PATENT APPLICATION

(11) **EP 1 723 968 A1**
(43) Date of publication of application: **22.11.2006**
(21) Application number: 04747144.6
(22) Date of filing: 01.07.2004
(51) Int. Cl.: A61K 45/00, A61K 31/195, A61K 33/00, A61P 9/08, A61P 9/00, A61P 9/10, A61P 25/00

(54) **ERYTHROCYTE FUNCTION MODIFYING SUBSTANCE**

(30) Priority: 29.01.2004 JP 2004022305
(71) Applicant: Keio University, Tokyo 108-8345 (JP)
(72) Inventor: SUEMATSU, Makoto, School of Medicine, Keio Uni., Tokyo 1608582 (JP)
(74) Representative: Woods, Geoffrey Corlett
(86) International application number: PCT/JP2004/009675
(87) International publication number: WO 2005/072773

(57) **Abstract**

The present invention provides a promoter for ATP release from erythrocytes, comprising a substance which stabilizes the structure of hemoglobin in erythrocytes in the T-state; a method of releasing ATP from erythrocytes using the promoter; an inhibitor against ATP release from erythrocytes, comprising a substance which stabilizes the structure of hemoglobin in erythrocytes in the R-state; and a method of inhibiting ATP release from erythrocytes using the inhibitor.

## Description

### TECHNICAL FIELD

The present invention relates to a regulator for ATP release from erythrocytes, comprising a substance which stabilizes the structure of hemoglobin in erythrocytes in the T-state or the R-state, and a pharmaceutical composition comprising the above substance.

### BACKGROUND ART

In scenes of disease treatment and clinical tests, development of a method of drug delivery to a specific tissue, (*i*.*e*., drug delivery system) is demanded in order to deliver a drug to a target site effectively and selectively. One of the important elements for establishing a drug delivery system is a vehicle for drugs. To date, numerous researches have been done concerning those substances which function as a vehicle for drugs. Among all, technology for using erythrocytes as a vehicle has been developed (Japanese Unexamined Patent Publications (Kohyo) Nos. 2003-522140 and 2001-512480). In the above publications, it is disclosed that erythrocytes bind to drugs or oxygen in their inside or on their surface and function as a carrier for transportation.

However, erythrocytes have only been recognized as a vehicle for transporting drugs or oxygen to a target site. No technology to artificially control the physiological functions of erythrocytes per se and to apply those functions to clinical scenes has been known yet. No methods have been found yet by which the oxygen release ability and vasodilatory effect of erythrocytes are manifested specifically at a target site.

On the other hand, it is known that erythrocytes release ATP in response to various stimulations such as decrease in blood oxygen partial pressure, decrease in pH, or mechanical deformation (Sprague, R. S. et al., Am. J. Physiol. Cell Physiol., 281, C1158-C1164, 2001). The ATP released from erythrocytes act on specific receptors on the surface of endothelial cells to thereby promote the synthesis and release of nitric oxide (NO) and prostacyclin in the vascular endothelium. It is known that both NO and prostacyclin are deeply involved in vasodilation reaction.

However, details of the molecular mechanism of ATP release from erythrocytes and the oxygen concentration dependency of the ATP release have not been elucidated. Therefore, how to specifically apply the ATP release phenomenon to the control of pathology has been still unclear.

### DISCLOSURE OF THE INVENTION

The present invention aims at providing erythrocytes which release ATP; erythrocytes comprising a drug which allows ATP release; and a pharmaceutical composition comprising erythrocytes which release ATP. Further, it is another object of the present invention to provide a method of quantitatively determining the amount of ATP released oxygen concentration dependently with erythrocytes.

As a result of extensive and intensive researches toward solution of the above problems, the present inventor has found that by stabilizing the structure of hemoglobin in the T-type or R-type, it is possible to regulate ATP release regardless of oxygen partial pressures or at desired oxygen concentrations. Thus, the present invention has been achieved.

The present invention relates to the following inventions.
(1) A promoter for ATP release from erythrocytes, comprising a substance which stabilizes the structure of hemoglobin in erythrocytes in the T-state.
   As a substance which stabilizes the structure of hemoglobin in erythrocytes in the T-state, at least one selected from the group consisting of bezafibrate, nitric oxide, carbon dioxide and adenosine may be given. The ATP release mentioned above may be performed under an oxygen partial pressure of 100 mmHg or less.
(2) A pharmaceutical composition comprising a substance which stabilizes the structure of hemoglobin in erythrocytes in the T-state.
   The substance which stabilizes the structure of hemoglobin in erythrocytes in the T-state and the oxygen partial pressure under which ATP release is performed are the same as described in (1) above. The pharmaceutical composition of the present invention may be used, for example, as a vasodilator or blood flow improver.
(3) A method of releasing ATP from erythrocytes, comprising stabilizing the structure of hemoglobin in the erythrocytes in the T-state.
   The ATP release may be performed under an oxygen partial pressure of 100 mmHg or less.
(4) An inhibitor against ATP release from erythrocytes, comprising a substance which stabilizes the structure of hemoglobin in erythrocytes in the R-state.
   As a substance which stabilizes the structure of hemoglobin in erythrocytes in the R-state, carbon monoxide or sulfonylurea may be given. The inhibition of ATP release may be performed under an oxygen partial pressure of 100 mmHg or less.
(5) A pharmaceutical composition comprising a substance which stabilizes the structure of hemoglobin in erythrocytes in the R-state.
   The substance which stabilizes the structure of hemoglobin in erythrocytes in the R-state and the oxygen partial pressure under which ATP release is inhibited are the same as described in (4) above. This pharmaceutical composition of the present invention may be used, for example, as a vasoconstrictor, blood flow regulator or vasodilation inhibitor.
(6) A method of inhibiting ATP release from erythrocytes, comprising stabilizing the structure of hemoglobin in the erythrocytes in the R-state.
   The inhibition of ATP release may be performed under an oxygen partial pressure of 100 mmHg or less.
(7) Erythrocytes in which the structure of hemoglobin is stabilized in the T-state.
   Examples of the above-described erythrocytes of the present invention include erythrocytes which have been treated with at least one substance selected from the group consisting of bezafibrate, nitric oxide, carbon dioxide, adenosine and hydrogen ion. ATP release may be performed under an oxygen partial pressure of 100 mmHg or less.
(8) Erythrocytes in which the structure of hemoglobin is stabilized in the R-state.
   Examples of the above-described erythrocytes of the present invention include erythrocytes which have been treated with carbon monoxide or sulfonylurea. Inhibition of ATP release may be performed under an oxygen partial pressure of 100 mmHg or less.
(9) A pharmaceutical composition comprising the erythrocytes of (7) above.
   The above-described pharmaceutical composition of the present invention may be used for treating ischemic diseases (for example, at least one selected from the group consisting of hemorrhagic shock, myocardial infarction, angina pectoris, cerebral infarction, intracerebral hemorrhage, obliterative arterial diseases, vascular disorders caused by diabetes, coronary artery stenosis, ischemic diseases of extremities, arteriosclerosis obliterans and ischemic ulcer/necrosis) or acidosis. The ischemic disease may be ischemia-reperfusion syndrome (for example, syndrome which results from at least one cause selected from the group consisting of post-shock resuscitation, perfusion after cold storage of organs, recanalization of blood flow after surgical operations, and reconstruction of obliterated blood vessels).
(10) A pharmaceutical composition comprising the erythrocytes of (8) above.
   The above-described pharmaceutical composition of the present invention may be used for treating vasodilatory diseases (for example, septic shock or anaphylactic shock).
(11) A method of measuring ATP, comprising quantitatively determining the amount of ATP released from erythrocytes in an oxygen concentration-dependent manner.
(12) A method of enhancing ATP release from erythrocytes, comprising adding adenosine to an erythrocyte suspension and exposing the resultant suspension to a no oxygen or low oxygen partial pressure condition.
   The no oxygen or low oxygen partial pressure condition is, for example, a condition where oxygen partial pressure is 0-150 mmHg. The concentration of adenosine is, for example, 0.1-10 µmol/L.
(13) A method of enhancing ATP release from erythrocytes, comprising adding adenosine to an erythrocyte suspension and exposing the resultant suspension to a carbon dioxide partial pressure of 60-80 mmHg.
   The concentration of adenosine is, for example, 0.1-10 µmol/L.
(14) A method of controlling ATP release from erythrocytes, comprising adding a substance that inhibits the anion permeation function of band 3 protein (*e.g*., sulfonylurea) to an adenosine-added erythrocyte suspension.
   As a specific example of sulfonylurea, 4,4'-diisothiocyanate-stilbene-2,2'-disulfonic acid (DIDS) may be given.
(15) A controller for ATP release from erythrocytes, comprising a substance that inhibits the anion permeation function of band 3 protein (e.g., sulfonylurea such as DIDS).

The controller of the present invention may be used, for example, to inhibit ATP release.

### BRIEF DESCRIPTION OF THE DRAWINGS

Fig. 1 is a graph showing relations between oxygen partial pressure and ATP release; and the erythrocyte release effects of saturating CO and bezafibrate.
Fig. 2 is a graph showing relations between oxygen partial pressure and ATP release; and the erythrocyte release effects of saturating CO and αNO-Hb.
Fig. 3 is graphs showing the functional sinusoidal density (FSD) of hepatic microcirculation, base excess and the recovery of bile secretion in rats measured 60 minutes after the following operations. 40% of the total body blood volume of the rats was collected, and then erythrocytes stabilized in the T-state were returned to them
Fig. 4 is a graph showing the results of measurement of ATP release when adenosine was added to extracellular fluid.
Fig. 5 is a graph showing the concentration-dependent, ATP release effect of adenosine.
Fig. 6 is graphs showing ATP release in the R-state.
Fig. 7 is graphs showing the inhibitory effect of DIDS on ATP release from carbon monoxide-treated erythrocytes.
Fig. 8 is graphs showing the dose-dependent effect of DIDS on ATP release.
Fig. 9 is graphs showing the ATP release inhibitory effect of DIDS under respective oxygen partial pressures.
Fig. 10 is graphs showing the carbon dioxide partial pressure-dependent increase of ATP release.

### BEST MODE FOR CARRYING OUT THE INVENTION

Hereinbelow, the present invention will be described in detail.

When tissues come under hypoxic conditions, adenosine triphosphate (ATP) is released from erythrocytes. The released ATP increases calcium concentrations via a specific receptor expressed on the surface of vascular endothelial cells to thereby promote the synthesis and release of nitric oxide (NO) and prostacyclin. This means that vasodilatory effect is produced by the above-mentioned NO and prostacyclin.

On the other hand, ATP released into plasma is degraded into adenosine diphosphate (ADP) and activates platelet aggregation and adhesion. This means that hemostasis occurs.

Thus, it is very important for the physiological function of the body that ATP is released from erythrocytes. However, relation between the oxygen concentration dependency of ATP release and the mechanism of physiological function have not been elucidated yet. Besides, no research has proved molecularly that erythrocytes possess the ability to release ATP (a vasodilatory substance) to thereby reach foci when they have reached low oxygen partial pressure sites.

Under circumstanced, the present inventor made intensive and extensive researches focusing on a point how ATP release from erythrocyte occurs under low oxygen partial pressures.

### 1. Relation between ATP Release and Hemoglobin Allostery

The pattern of ATP release from erythrocytes drew a sigmoid curve similar to an oxygen dissociation curve in relation to oxygen partial pressure. The oxygen dissociation curve means to a curve showing the rate of oxygen-bound hemoglobin at various partial pressures of oxygen. This ATP release pattern depends on hemoglobin (Hb) allostery in erythrocytes and resembled the pattern of Hb sigmoid curve. Then, the present inventor considered that ATP release reaction in erythrocytes is oxygen dependent because hemoglobin perceives oxygen concentration and causes this phenomenon. The present inventor measured ATP release from erythrocytes after stabilizing the structure of hemoglobin in the T- or R-configuration. The results revealed that the R-configuration inhibits ATP release and the T-configuration activates ATP release. Thus, the above-described observation of the inventor was proved.

Hemoglobin is a protein with a molecular weight of 64,500 which functions as a vehicle for oxygen. The oxygen binding affinity of hemoglobin is related to the structure of hemoglobin. Briefly, hemoglobin takes the T-configuration (called the "T-state") under low oxygen partial pressures, and takes the R-configuration (called the "R-state") under high oxygen partial pressures. The term "T-state" used herein refers to a structure where the heme is 5-coordinate and which has low affinity to oxygen (oxygen is easily released at the peripherals). The term "R-state" used herein refers to a structure where the heme is 6-coordinate and which has high affinity to oxygen (oxygen is not easily released at the peripherals).

Several drugs which stabilize the structure of hemoglobin in the T- or R-state have been found. In order to examine ATP release patterns when hemoglobin allostery is stabilized in the T- or R-state with these drugs, an NO derivative which was known to stabilize hemoglobin in the T-state was used (Yonetani, T. et al., J. Biol. Chem., 273(32), 20323-20333(1998)).

When this NO derivative was added to hemoglobin in the absence of oxygen, erythrocytes released ATP constantly regardless of the oxygen partial pressure. On the other hand, when similar measurement was conducted using carbon monoxide (CO) which is known to stabilize hemoglobin in the R-state, erythrocytes released little ATP regardless of the oxygen partial pressure. Thus, the inventor has found that it is possible to regulate ATP release regardless of the oxygen partial pressure when the structure of hemoglobin is stabilized in the T- or R-state. This means that when the structure of hemoglobin shifts to the T-state because of the lowering of blood oxygen partial pressure, or when the structure of hemoglobin is stabilized in the T-state, ATP (that has vasodilatory effect) can be released from erythrocytes effectively. Further, this means that when the structure of hemoglobin shifts to the R-state because of the increasing of blood oxygen partial pressure, or when the structure of hemoglobin is stabilized in the R-state, ATP release from erythrocytes can be inhibited.

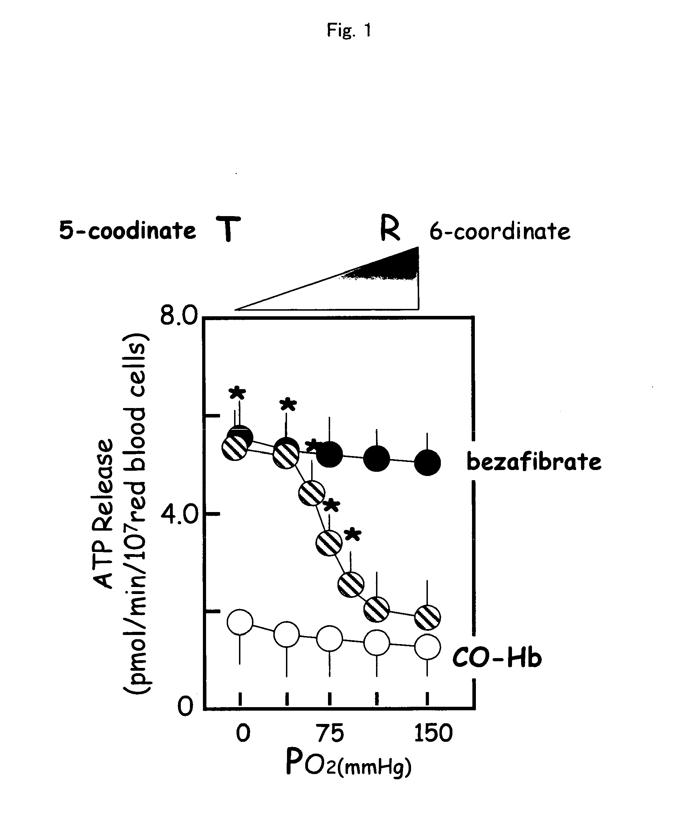

As shown in Fig. 1, human erythrocytes release ATP extracellularly when the oxygen partial pressure decreases. Since this ability shows a sigmoid curve with a border (flexion point) at an oxygen partial pressure of 50-70 mmHg, the present inventor presumed that hemoglobin performs the sensing of oxygen concentration. When erythrocytes were saturated with CO that stabilizes the structure of hemoglobin in the so-called R-state (heme 6-coordinate), this ability was completely eliminated. On the other hand, when erythrocytes were treated with bezafibrate (100 µmol/L, 30 min) that stabilizes the structure of hemoglobin in the so-called T-state (heme 5-coordinate), the treated erythrocytes released a large quantity of ATP even at normal oxygen concentration.

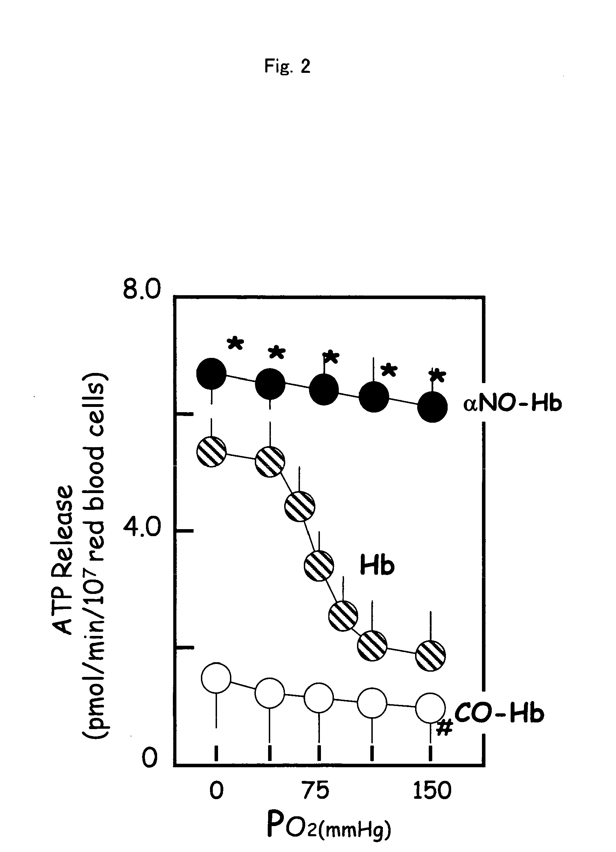

Further, as shown in Fig. 2, when NO that stabilizes hemoglobin in the T-state was added to erythrocytes in 0.5 equivalents to hemoglobin, the resultant erythrocytes released ATP constantly regardless of the oxygen concentration (see the curve "αNO-Hb").

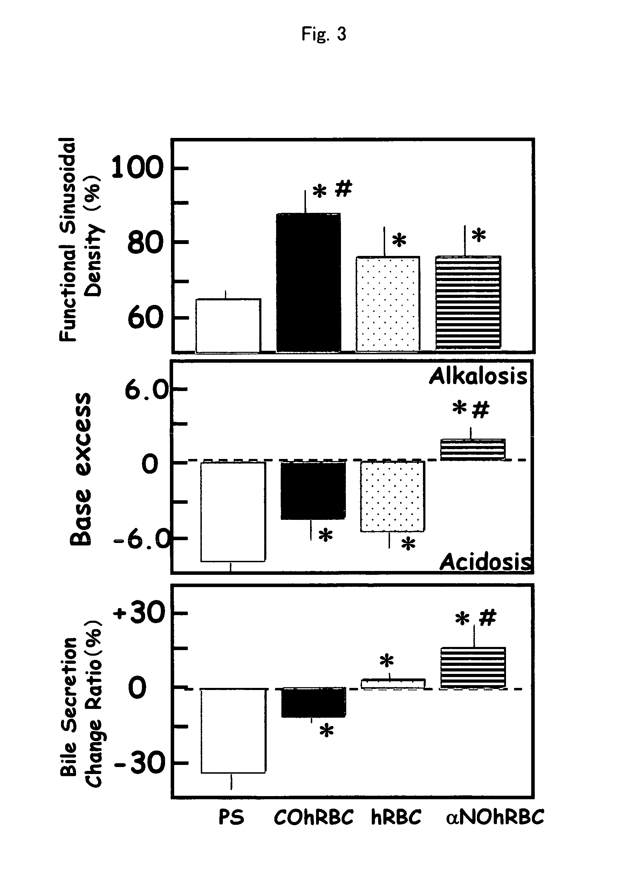

Therefore, it is believed that erythrocytes in which the structure of hemoglobin is stabilized in the T-state are effective in ameliorating ischemia-reoxidation disorders. Specific examples of organs or tissues where ischemia-reperfusion disorders may occur include, but are not limited to, heart, liver, brain, lung, kidney, blood vessel, skeletal muscle, spleen, stomach, small intestine and colon. In particular, in ischemia-reperfusion disorders in the liver, erythrocytes reveal an effect of dramatically improving the recovery of bile secretion. This effect has become clear in an experiment (described herein later) in which the dynamics of hepatic microcirculation was examined by collecting 40% of the total body blood volume of rats and returning to the rats the equal amount of hemoglobin in the form of erythrocytes or stabilized erythrocytes in the T-state 15 minutes after the exsanguination. Briefly, the results revealed that the effect obtained when erythrocytes stabilized in the T-state had been returned is superior to the effect obtained when normal erythrocytes had been returned in terms of functional sinusoidal density (FSD), improvement of acidosis, and recovery ratio of bile secretion (Fig. 3).

Generally, donated blood is stored at 4°C. Under such low temperature storage, hemoglobin comes into the R-state without any treatment. Further, when hemoglobin is converted to carboxyhemoglobin (COHb) during low temperature storage, it is possible to inhibit ATP release and to thereby retain the energy of erythrocytes. When a T-state stabilizing substance was added to erythrocytes in such R-sate, ATP release was activated. Thus, ATP release recovery effect was also recognized even in erythrocytes in the R-state or CO-saturated erythrocytes. Further, in the ischemia-reperfusion disorders in the liver described above, it was shown that functional sinusoidal density could be recovered to 90% relative to the density before shock, with CO-saturated erythrocytes (Fig. 3). This means that erythrocytes may be used as a carrier which transports CO to peripheral tissues.

From the above, it could be said that a drug regulating the T-R transition of hemoglobin not only initiates the oxygen release ability of erythrocytes but also has the ability to allow erythrocytes to release ATP in response to the lowering of oxygen partial pressure which may occur in resistance vessels of the body. Therefore, every drug that is capable of altering the T-R transition of hemoglobin, and erythrocytes treated with such a drug are encompassed in the present invention. Such erythrocytes may be used in "value-added blood transfusion" as a value-added blood capable of activating ATP release.

ATP acts on vascular endothelial cells in the body, increases NO and enhances blood flow. In the liver, ATP acts on the hepatic cell membrane to thereby activate the rhythmical contraction of bile canaliculi, which results in activation of bile secretion. Further, since T-state stabilizing substances have a biological effect which erythrocytes manifest when they reach low oxygen environments (i.e., a biological effect of improving shock by enhancing oxygen release and vasodilation), they may be used as a peripheral circulation improving drug.

### 2. Stabilization of the T-State or R-State

### (1) Stabilizing Substances

In the present invention, specific examples of substances which stabilize hemoglobin in the T-state to thereby allow ATP to be released from erythrocytes (hereinafter, called the "T-state stabilizing substance") include bezafibrate, NO, NO derivatives, carbon dioxide, adenosine and hydrogen ion. At least one of these substances may be used alone or in appropriate combination. It should be noted that the T-state stabilizing substance is not limited to those substances enumerated above.

The T-state stabilizing substance may be a partially modified substance or derivative of any of the above-mentioned substances as long as it has an effect of allowing ATP to be released from erythrocytes.

Methods of stabilizing hemoglobin in the T-state are known (Kilmartin J V, Rossi-Bernardi L, "Interaction of hemoglobin with hydrogen ions, carbon dioxide, and organic phosphates", Physiological Review, vol.53, 836-890 (1973)). For example, hemoglobin may be stabilized in the T-state by treating with carbon dioxide, ATP, inositol hexakisphosphate, hydrogen ion (H⁺), chloride ion (Cl⁻) or the like.

Specific examples of substances which stabilize hemoglobin in the R-state to thereby inhibit ATP release from erythrocytes (hereinafter, called the "R-state stabilizing substance") include carbon monoxide (CO) and sulfonylurea. It should be noted that the R-state stabilizing substance is not limited to the substances given above.

### (2) ATP Release or Inhibition Thereof

Erythrocytes in which hemoglobin is stabilized in the T-state as described above have a function of releasing ATP. The term "releasing ATP" means that an ATP release curve does not take a usual sigmoid curve observed in hemoglobin in relation to oxygen partial pressure and that more ATP than the amount of ATP release shown in the above-described usual sigmoid curve is released at least under oxygen partial pressures above the flexion point (e.g., 100 mmHg PO₂) (see, for example, the curve "bezafibrate" in Fig. 1). Accordingly, as long as such an ATP release curve is drawn, the amount of ATP release may be equal to, more than or less than the amount of ATP release shown in the above-described usual sigmoid curve under oxygen partial pressures below the flexion point of the usual sigmoid curve. However, in the present invention, promotion of ATP release is performed under an oxygen partial pressure of 100 mmHg or less, preferably 40-80 mmHg.

On the other hand, erythrocytes in which hemoglobin is stabilized in the R-state as described above have a function of inhibiting ATP release. The term "inhibiting ATP release" means that an ATP release curve does not take a usual sigmoid curve observed in hemoglobin in relation to oxygen partial pressure and that less ATP than the amount of ATP release shown in the above-described usual sigmoid curve is released at least under oxygen partial pressures below the flexion point (see, for example, the curve "CO-Hb" in Fig. 1). Accordingly, as long as such an ATP release curve is drawn, the amount of ATP release may be equal to, less than or more than the amount of ATP release shown in the above-described usual sigmoid curve under oxygen partial pressures above the flexion point of the usual sigmoid curve. However, in the present invention, inhibition of ATP release is performed under an oxygen partial pressure of 100 mmHg or less, preferably 40-80 mmHg. The oxygen partial pressure in the arterioles is 40-80 mmHg.

### 3. Relation between Adenosine Addition and ATP Release

### (1) Relation with Oxygen Partial Pressure

Adenosine is a nucleoside containing adenine (a purine derivative) in its base moiety and serves as a raw material for ATP.

Since adenosine is taken into cells via a transport system passing through cells, the present inventor contemplated to add adenosine to an erythrocyte suspension.

As a result, it was found that addition of adenosine to the extracellular fluid enhances ATP release under a no oxygen or low oxygen condition. Especially, when 1 µmol/L of adenosine was added to the extracellular fluid and reacted for 5 minutes and then the fluid was exposed to a no oxygen or low oxygen condition for 1 minute, ATP release was enhanced about two times.

This means that a conserved blood with high ATP release ability can be obtained by adding adenosine to, for example, blood for transfusion. Adenosine-added blood is extremely useful because such blood may be used effectively in emergency medical service and at the time of operation as a transfusion product.

In the present invention, adenosine is added to an erythrocyte suspension at a rate of 0.1-10 µmol/L, preferably 1-5µmol/L.

The term "no oxygen condition" used herein means that oxygen partial pressure is 0 mmHg, and the term "low oxygen condition" used herein means a condition where oxygen partial pressure is more than 0 mmHg and less than the normal oxygen partial pressure (150 mmHg, in air).

By exposing the erythrocyte suspension to the above-described oxygen partial pressure for 10-60 minutes, it is possible to enhance ATP release.

### (2) Relation with Carbon Dioxide Partial Pressure

It is known that carbon dioxide (CO₂) has an effect called "hypercapnic protection". Briefly, when CO₂ partial pressure is increased by artificially reducing ventilation with mechanical respiration, the prognosis of ARDS (adult respiratory distress syndrome) is improved. The mechanism of this effect has not yet been elucidated.

In the present invention, it has become clear that ATP release can be enhanced by changing CO₂ partial pressure under normal oxygen partial pressure (Normoxia). CO₂ partial pressure is preferably 60-80 mmHg.

### (3) Relation with Anion Exchanger

A transmembrane glycoprotein called "Band III" (band 3) exists in the erythrocyte membrane (about 10⁶ molecules/erythrocyte). This protein is composed of two functional domains; one is a hydrophilic domain on the N-terminal side and the other is a domain on the C-terminal side. The N-terminal hydrophilic domain, which is attached to the cytoskeleton via ankyrin, is involved in the maintenance of erythrocyte morphology The C-terminal domain is an intramembrane domain which spans the erythrocyte membrane 14 times and is responsible for anion permeation function (that is a function of mediating the exchange reaction between Cl⁻ and HCO₃⁻ and transporting and discharging CO₂ gas). A membrane protein in charge of this function is called "anion exchanger".

Since the present inventor has found that extracellular ATP release is performed through the above-described anion exchanger, the inventor considered that it would be possible to control the amount of ATP release by increasing the resistance of the above-described anion exchanger to thereby inhibit its function (anion permeation function).

The substance used for the above-described control of ATP release is not particularly limited as long as the substance (drug or low molecular weight compound) binds to the extracellular domain of Band III to thereby inhibit its anion permeation function.

Specific examples of drugs that bind to the extracellular domain of Band III include DIDS (4,4'-diisothiocyanate-stilbene-2,2'-disulfonic acid) which is a sulfonylurea used in treating diabetes. DIDS is preferably used. By adding a substance that binds to the extracellular domain of Band III to an erythrocyte suspension, it is possible to control the amount of ATP release.

For example, DIDS is preferably added to an erythrocyte suspension to give a concentration of 1x10⁻⁶ - 1x10⁻⁴ mol/L

The reactions described in (1) and (2) above are controlled by a substance that binds to the extracellular domain of Band III. Therefore, those skilled in the art can control the amount of ATP release appropriately taking into account of oxygen partial pressure and carbon dioxide partial pressure.

### 4. Pharmaceutical Compositions

In the present invention, substances which stabilize the structure of hemoglobin in erythrocytes in the T-state may be used as a pharmaceutical composition such as vasodilator or blood flow improver because those substances promote ATP release from erythrocytes.

On the other hand, substances which stabilize the structure of hemoglobin in erythrocytes in the R-state may be used as a vasoconstrictor, blood flow regulator or vasodilation inhibitor because those substances inhibit ATP release from erythrocytes.

The above-described substance which stabilizes in the T-state and the substance which stabilizes in the R-state are called "T-R transition regulators" in the present invention.

Further, the present invention provides erythrocytes treated with the T-R transition regulator. Since the erythrocytes of the invention are regulated in the T- or R-state so that they are capable of promoting or inhibiting ATP release, it is possible to use the erythrocytes as a pharmaceutical composition.

Erythrocytes which are the resource of the pharmaceutical compositions of the invention are preferably conserved donated blood or blood for autologous transfusion, but are not limited to these bloods.

### (1) T-R Transition Regulators

When the T-R transition regulating drug of the invention is used as an ATP release promoter or ATP release inhibitor, the drug may be administered systemically by conventional intravenous or intraarterial administration. Alternatively, the drug may be administered locally through postoperative, reconstructed vessels.

Therefore, a method of administration combined with catheter techniques or surgical operations may also be used.

Substances which stabilize in the T-state contribute to vasodilation or improvement of blood flow. Therefore, target patients to be administered the substance which stabilize in the T-state are patients with, for example, ischemic disorders or cardiovascular diseases. Specific examples of these diseases include hemorrhagic shock, myocardial infarction, angina pectoris, cerebral infarction, intracerebral hemorrhage, obliterative arterial diseases, vascular disorders caused by diabetes, coronary artery stenosis, ischemic diseases of extremities, arteriosclerosis obliterans, ischemic ulcer/necrosis, ischemia-reperfusion syndrome (syndrome resulting from post-shock resuscitation, perfusion after cold storage of organs, recanalization of blood flow after surgical operations, reconstruction of obliterated blood vessels, and so on), ischemic heart diseases, gastrointestinal hemorrhage, DIC (disseminated intravascular coagulation), traumatic shock and multiple organ failure. Further, substances which stabilize in the T-state may be used for treating or improving acidosis or shock.

Substances which stabilize in the R-state contribute to vasoconstriction or inhibition of blood flow. Therefore, target patients to be administered the substance which stabilize in the R-state are patients with vasodilatory diseases, for example, sepsis, anaphylactic shock or endotoxin shock.

Dose levels of the above-described drug may vary depending on the age, sex and symptoms of the patient to be treated, the administration route, the number of times of administration and the form of preparation. For example, in the treatment of hemorrhagic shock, 800 ml of a drug preparation (containing the drug at 10 g/dl) may be administered per administration one or several times a day. The drug preparation may be administered 3 to 4 times a day.

It is also possible to add the T-R transition regulator of the invention to blood taken out of the body for the purpose of transfusion. In this case, 5-10 ml of the regulator may be added per 200 ml of blood (blood Hb concentration: 10 g/dl).

### (2) Erythrocyte Preparations Treated with the T-R Transition Regulating Drug

### (2-1) Hemoglobin Allostery

Research and development of oxygen infusions necessary for emergency life-saving have mainly developed preparations by purifying hemoglobin from donated blood remaining after expire date and modifying or enclosing the resultant hemoglobin with a biocompatible material.

Due to recent studies, a possibility is being revealed. That is, erythrocytes perceive intravascular oxygen concentration and actively release or recover low molecular weight gasses such as NO or organic acids; a part of such gasses or acids may be involved in vasodilation or control of platelet activation. This shows a possibility that erythrocytes are not only involved in oxygen transport and regulation of acid-base equilibrium, but also positively maintain microcirculation blood flow. In other words, these value-added functions which erythrocytes essentially possess cannot be supplemented completely with artificial oxygen transporter with conventional purified hemoglobin modified products. Elucidation of the molecular mechanism showing that such erythrocytes as a sink reservoir for vascular effectors are involved in the control of vascular functions is extremely important, especially when application of shock treatment to emergency life-saving is contemplated. It is indispensable to develop a novel erythrocyte preparation provided with high oxygen transport ability and microcirculation blood flow maintenance function while maintaining the cell membrane function.

The present invention develops improvement of erythrocyte conservation technology using the technology of protein function modification with highly membrane permeable low molecular weight gasses (e.g., NO, CO), as well as effective use of donated blood from volunteers. Specifically, the invention aims at developing a practical method for creating an erythrocyte preparation using αNO-Hb (hemoglobin fixed with NO) or CO-Hb (hemoglobin fixed with CO). In this erythrocyte preparation, less hemoglobin is capable of transporting oxygen equivalent in amount to the oxygen possessed by abundant hemoglobin. The invention also aims at developing a novel method of conserving erythrocytes. It is an object of the invention to put these products into practical use.

In the present invention, the advantage that the burden of heme detoxification can be reduced in individuals who are administered the erythrocyte preparation should also be noted. Different from normal individuals, those individuals after surgical operation or under shock state undergo abnormal enhancement of the degradation of heme to bilirubin because heme oxygenase (a heme detoxification enzyme) is induced due to cytokinemia or hypoxic stress (Kyokane T, Norimizu S, Taniai H, Yamaguchi T, Takeoka S, Tsuchida E, Naito M, Nimura Y, Ishimura Y, Suematsu M. Gastroenterology 120, 1227-1240 (2001)). Therefore, decrease in the absolute amount of heme per the amount of oxygen transport is desirable not only from the viewpoint of saving of blood resource, but also in view of reduction of the risk of hyperbilirubinemia in the individuals who are administered the erythrocyte preparation.

It is also possible to apply the novel NO-Hb as an oxidized flush solution for grafts for implantation. Usually, the affinity between Hb heme and molecular oxygen increases under a low temperature (4 °C) condition. Therefore, αNO-Hb is suitable for improving oxygen transport at the time of reperfusion. On the contrary, in order to reduce the oxygen paradox occurring at the time of reperfusion, CO-Hb may be used. Further, CO which is not a radical molecule may be applicable as a mild vasorelaxant. Vasodilatory effect by Hb-CO can be expected in muscles and the brain where heme proteins with higher CO affinity than Hb exist. These possibilities can also be led to creation of new demands for remaining erythrocytes through the experimental evaluation made in the present invention. To carry out the effective use of αNO-RBC and CO-RBC proposed by the present invention is extremely important from the viewpoint of improving the quality of emergency life-saving and also in view of effective use of the precious blood resource obtained from people's charity.

In order to develop a value-added erythrocyte preparation by Hb allostery modification, the following (i) and (ii) must be done. (i) The hypothesis that erythrocytes occupying 50% of the blood volume work not only as a mere oxygen transporter but also as a metabolic sink that releases vasodilatory substances or absorbs vasoconstricting substances upon perception of changes in oxygen partial pressure and shear stress in microcirculation regions (especially, that Hb allostery changes resulted from oxygen desorption or the like control this function) should be examined, and the mechanism thereof should be elucidated. (ii) This concept should be introduced into an artificial oxygen transporter, and commercialization of such a product should be aimed. In order to verify the contents described in (i) above, the effect ofNO-Hb or CO-Hb on resuscitation from hemorrhagic shock is examined in the present invention. Further, in order to examine the contents described in (ii) above, Hb modified erythrocytes are applied to experimental animals in the present invention.

### (i) Examination of the Effect of αNO-Hb or CO-Hb on Resuscitation from Hemorrhagic Shock

According to Wigger's hemorrhagic shock protocol, 40% exsanguination shock was initiated in rats, followed by trial of resuscitation with erythrocytes having αNO-Hb or CO-Hb (these erythrocytes were isolated and prepared from allogeneic rats). Recovery effects were examined comparatively using a biomicroscopic analysis system for hepatic microcirculation. Comparative study was made using other indicators than oxygen supply in the microcirculation; other indicators used were functional capillary density (FCD), leukocytes adhesion, Kupffer cell activation, and so forth. Further, 40% exsanguination shock was initiated in rats according to Wigger's hemorrhagic shock protocol, followed by trial of resuscitation with human erythrocytes having αNO-Hb or CO-Hb (these erythrocytes were isolated and prepared from human peripheral blood).

It has been well known for a long time that the oxygen dissociation curve shows a right (or left) shift because of the presence of biomolecules such as 2,3-DPG or synthetic compounds. Gaseous molecules such as NO and CO which are excellent in membrane permeability and bind to the heme of Hb are also Hb allostery regulating molecules.

### (ii) Application of Hb Modified Erythrocytes to Experimental Animals

The results of initiation of 40% exsanguination shock according to Wigger's hemorrhagic shock protocol and trial of resuscitation with αNO-RBC confirmed that αNO-RBC improves patency at the capillary vessel level remarkably and restores bile secretion remarkably. Further, an improving effect similar to the effect seen in normal erythrocytes (not binding to CO) were also confirmed in CO saturated erythrocytes which are believed to have no oxygen transport ability by nature. In the examination using human peripheral blood, it was confirmed that αNO-RBC, CO saturated erythrocytes and normal erythrocytes have an improving effect on systemic acidosis.

### (2-2) Erythrocytes Stabilized in the T-State

From what have been described above, the erythrocytes stabilized in the T-state prepared in the present invention may be used as a pharmaceutical composition for vasodilation and blood flow improvement. Further, the erythrocytes prepared in the present invention may be used as a pharmaceutical composition for treating and improving ischemic diseases, acidosis and shock.

Erythrocytes used in the present invention may be collected from the recipient individual to be treated so that the resultant erythrocyte preparation is immuno-compatible. Alternatively, donated blood from volunteers may be used. The erythrocytes used in the present invention are mammal erythrocytes, preferably human erythrocytes.

A pharmaceutical composition comprising the erythrocytes of the invention may be used for the specific purposes of treating, preventing or testing diseases occurring, for example, in the following tissues.
Gastrointestinal system: oral cavity, pharynx, esophagus, stomach, small intestine, colon, liver, pancreas, etc.
Respiratory system: tracheal tube, bronchial tube, lung, etc.
Urinary system: kidney, bladder, etc.
Cardiovascular system: heart, artery, vein
Lymphatic system: lymphatic vessel, lymph node, spleen, thymus
Central nervous system: brain (cerebrum, diencephalon, midbrain, cerebellum), medulla oblongata, spinal cord
Muscle: skeletal muscle, smooth muscle, etc.

These diseases may be a single disease, a combination of some of diseases occurring simultaneously, or a disease occurring simultaneously with other diseases than those described above; any of them may be a target of the pharmaceutical composition of the invention. In the present invention, it is preferable to use the pharmaceutical composition for the purpose of vasodilation, improvement of blood flow or improvement of shock.

In the present invention, it is possible to use erythrocytes containing a drug which allow ATP to be released as a pharmaceutical composition for treating ischemic diseases or cardiovascular diseases.

Specific examples of ischemic diseases and cardiovascular diseases include, but are not limited to, hemorrhagic shock, myocardial infarction, angina pectoris, cerebral infarction, intracerebral hemorrhage, obliterative arterial diseases, vascular disorders caused by diabetes; or coronary artery stenosis, ischemic diseases of extremities, arteriosclerosis obliterans, ischemic ulcer/necrosis, ischemia-reperfusion syndrome, ischemic heart diseases, gastrointestinal hemorrhage, DIC (disseminated intravascular coagulation), traumatic shock and multiple organ failure. As the ischemia-reperfusion syndrome, syndrome resulting from at least one cause selected from the group consisting of post-shock resuscitation, perfusion after cold storage of organs, recanalization of blood flow after surgical operations, and reconstruction of obliterated blood vessels may be given. The pharmaceutical composition of the present invention may be used for treating these diseases; they have an effect as a blood flow improver or vasodilator.

Further, the pharmaceutical composition of the present invention may be used as a therapeutic for acid-base equilibrium disorders (e.g., acidosis) or as a shock improver.

Erythrocytes release ATP extracellularly via the allostery change in hemoglobin caused by changes in oxygen concentration as described above. Specifically, erythrocytes are apt to release ATP in low oxygen states, and are not apt to release ATP in oxygen-saturated states.

Therefore, in those sites where blood flow stagnates (*i.e.,* sites where the volume of blood pathway has been reduced or eliminated because of thrombi, vascular hyperplasia, vasoconstriction, vascular obliteration, etc.), fresh blood containing sufficient oxygen can not be supplied. Thus, the blood in such a site comes under a low oxygen state. Hemorrhagic shock also induces a low oxygen state. Further, in the central part of cancer cells, oxygen supply is not sufficient because the vascular system is undeveloped, and cancer cells are under low oxygen states. This causes a problem that oxygen radicals are not produced efficiently in radiotherapy, and thus sufficient therapeutic effect cannot be obtained. Further, since cells need abundant oxygen, severe conditions such as cerebral infarction or myocardial infarction may possibly occur when oxygen supply is stopped (e.g., when blood flow is cut off).

The erythrocytes of the present invention enable organ blood flow to be maintained in such a low oxygen state by supplying ATP effectively.

In examination and/or treatment of the intestine with a colonoscope, the intestine is swollen with air. Thus, the pressure added to the intestine prevents blood flow, exposing enterocytes to a low oxygen state. Here, by allowing the gas used to include abundant carbon dioxide, the erythrocytes of the invention are capable of supplying ATP to thereby improve the low oxygen state of the intestine.

The ATP produced in erythrocytes and released therefrom act on a specific receptor present in vascular endothelial cells and increase NO and prostacyclin to thereby relax and dilate blood vessels. Therefore, ATP is capable of improving blood flow by relaxing and dilating blood vessels in which blood flow is stagnating.

Further, the released ATP is rapidly degraded into ADP in the blood. The thus produced ADP causes platelet aggregation via a specific receptor present in platelets. Therefore, a site showing a low oxygen state resulting from hemorrhage can be effectively stanched by the above-described mechanism.

Dose levels of the pharmaceutical composition of the invention may vary depending on the age, sex and symptoms of the patient to be treated, the administration route, the number of times of administration and the form of preparation. For example, in the treatment of hemorrhagic shock after surgery for liver disease, approximately 200-800 ml of a concentrated pack containing hemoglobin at 20 g/dl may be administered per administration one or several times a day The pharmaceutical composition may be administered 3 to 4 times a day

### (2-3) Erythrocytes Stabilized in the R-State

The erythrocytes stabilized in the R-state prepared in the present invention may be used as a pharmaceutical composition for treating disease with significant systemic vasodilation (vasodilatory diseases) such as septic shock or anaphylactic shock.

As described in (2-2) above, the pharmaceutical composition containing erythrocytes stabilized in the T-state is effective in treating hemorrhagic shock. This means that it is possible to use either erythrocytes stabilized in the T-state or erythrocytes stabilized in the R-state depending on the shock state to be treated.

Dose levels of the pharmaceutical composition of the invention may vary depending on the age, sex and symptoms of the patient to be treated, the administration route, the number of times of administration and the form of preparation. For example, in the treatment of septic shock or anaphylactic shock, approximately 200-800 ml of a concentrated pack containing hemoglobin at 20 g/dl may be administered per administration one or several times a day The pharmaceutical composition may be administered 3 to 4 times a day

### 5. Method of Measuring Blood Oxygen Content

According to the present invention, it has become clear that the relation between oxygen concentration and ATP release in erythrocytes draws a sigmoid curve similar to the oxygen dissociation curve of hemoglobin. Therefore, in the present invention, it is possible to indirectly measure the reserve ability of erythrocyte function by measuring the amount of hypoxic ATP release.

The method of the invention of measuring blood oxygen content is effective in clinical tests and treatment scenes. With the measuring method of the invention, measurement of oxygen content or test of blood contamination in blood for transfusion can be performed simply.

Blood to be used in this method is a blood sample taken from a subject for oxygen content measurement. It is desirable to measure ATP promptly after the collection of the sample. The amount of this blood sample to be taken is 5-400 ml, preferably 5-10 ml.

For measuring ATP in blood, a blood sample may be applied to a commercial measuring instrument.

It is possible to determine the oxygen content of a sample by obtaining a relation formula between oxygen content and the amount of blood ATP in advance and substituting the amount of blood ATP obtained by measurement into the formula.

Hereinbelow, the present invention will be described more specifically with reference to the following Examples. However, the present invention is not limited to these Examples.

### EXAMPLE 1

Human peripheral blood sample (20 ml) was taken, heparinized and centrifuged at 2000 rpm for 7 minutes. The erythrocyte pellet was collected and washed. Then, an erythrocyte suspension (1x10⁷ cells/ml) was prepared with Krebs buffer. A tightly sealed cuvette containing 1.8 ml of Krebs buffer in which oxygen concentration is adjusted at a desired level within a range of 0-150 mmHg was incubated at 37°C. A 200 µl aliquot of the erythrocyte suspension was injected into the tightly sealed cuvette with a microsyringe. After 5 minutes, the cuvette was placed in ice at 4°C to terminate the reaction. A 200 µl sample was taken while it was still 4°C, and centrifuged. The ATP in the resultant supernatant was quantitatively determined by luciferin assay. Normal erythrocytes drew a curve with a flexion point at around 50-70 mmHg in oxygen partial pressure (PO₂) (the sigmoid curve shown in Fig. 1). CO-saturated erythrocytes prepared by feeding 100% CO gas to erythrocytes for one minute showed a constant ATP release at a low level (the curve "CO-Hb" in Fig. 1), and erythrocytes treated with bezafibrate at 10⁻⁴ mol/L (final concentration) showed a constant ATP release at a high level (the curve "bezafibrate" in Fig. 1). Mark "*" in Fig. 1 indicates that there is a significant difference (P<0.05) from the measured value under normal oxygen partial pressure (150 mmHg, in air).

### EXAMPLE 2

In this example, the effects of carbon monoxide-saturated erythrocytes (CO-Hb) and NO treated erythrocytes (αNO-Hb) on ATP release were examined. The method was as described in Example 1 above. In order to examine the effect of NO, an experiment was conducted using erythrocytes prepared as described below. Briefly, collected erythrocytes were completely deoxygenated by feeding argon gas. Then, NO-glutathione was added thereto so that the concentration of this substance to the concentration of Hb was 1:2. Several minutes after the addition, the erythrocytes were centrifuged and recovered. The hemoglobin contained in the above erythrocytes is expressed as "αNO-Hb".

Normal Hb and CO-Hb showed results similar to those shown in Example 1 (Fig. 2). αNO-Hb showed a constant ATP release at a sill higher level than the "bezafibrate" shown in Example 1 (Fig. 2). Mark "*" in Fig. 2 indicates that there is a significant difference (P<0.05) from normal erythrocytes (Hb).

### EXAMPLE 3

Recovery Effect of Human Washed Erythrocyte Sample upon Hepatic Dysfunction Initiated by 40% Exsanguination Shock

Wistar male rats (250-300 g) were anesthetized by intramuscularly injecting 50 mg/kg of pentobarbital sodium. The tracheal tube of each of the anesthetized rat was opened, and a catheter was inserted into the femoral artery. Further, a catheter was inserted into the common bile duct to monitor the bile secretion. The left carotid artery was also cannulated to form an exsanguination line. Exsanguination was performed at 1-2 ml/min until the total blood exsanguinated reached 40% of the total body blood volume. Fifteen minutes under shock state after the completion of exsanguination, each preparation was administered to the rat in such a manner that a specific amount of Hb equivalent to the lost blood was administered. Sixty minutes thereafter, base excess was calculated from hepatic microcirculation, bile flow volume, and the Hb concentration and bicarborate concentration pH in the arterial blood (Fig. 3). Usually, base excess values +2.5 or above mean alkalosis and values -2.5 or below mean acidosis. In Fig. 3, PS shows the results of resuscitation with physiological saline in an amount equivalent to the amount of lost blood (n=7); COhRBC shows the results of resuscitation with CO saturated human erythrocytes (n=5); hRBC shows the results of resuscitation with air saturated human erythrocytes (n=5); and αNOhRBC shows the results of resuscitation with human erythrocytes which have Hb whose *α* subunit is saturated with NO (n=7). Further, in Fig. 3, mark "*" represents *P<0.05 (significantly different from PS group) and mark "#" represents *P<0.05 (significantly different from hRBC group). Dynamics of hepatic microcirculation were recorded with the video-enhanced, inverted biomicroscopic system previously reported.

When individual erythrocyte preparations were administered, the density of blood vessels in which sinusoidal blood flow in charge of hepatic microcirculation was maintained was remarkably improved in any of the groups treated with the three erythrocyte preparations (upper panel in Fig. 3). In particular, when CO-saturated erythrocytes (COhRBC) which are believed to be incapable of transporting oxygen was used, the functional sinusoidal density of the liver was recovered up to about 90% of the pre-shock density This result does not contradict the sinusoidal blood flow maintaining effect of CO in *ex vivo* liver which the present inventor has previously reported (Suematsu M, Goda N, Sano T, Kashiwagi S, Egawa T, Shinoda Y, and Ishimura Y, "Carbon monoxide: an endogenous modulator of sinusoidal tone in the perfused rat liver", J Clin Invest. 1995 Nov; 96(5): 2431-2437). The above result made it clear for the first time that erythrocytes can be used as a carrier for delivering CO to peripheral tissues.

On the other hand, for the purpose of examining improving effects on systemic acidosis, base excess was calculated from the Hb concentration and bicarborate concentration pH in the arterial blood. Usually, base excess values +2.5 or above mean alkalosis and values -2.5 or below mean acidosis. All the three erythrocyte preparations revealed a significant improving effect in base excess. αNOhRBC showed the highest improving effect with a significant difference from COhRBC and hRBC (#P<0.05).

In addition to these improving effects, improving effects on bile secretion (an indicator of viability of the liver as a whole) were also observed. The intensity of the effect was in the following order: αNOhRBC > hRBC > COhRBC. In particular, αNOhRBC revealed a significant bile secretion enhancing effect even compared to the pre-shock bile secretion. It has been *shown in vitro* that ATP causes activation of the rhythmical contraction of bile canaliculi (Kitamura T, Brauneis U, Gatmaitan Z, Arias IM, "Extracellular ATP, intracellular calcium and canalicular contraction in rat hepatocyte doublets", Hepatology. 1991 Oct; 14(4 Pt 1): 640-647). The result that αNOhRBC with high ATP secretion ability showed bile secretion activating effect does not contradict this fact. Anyway, it was demonstrated that administration of CO-hRBC is useful as a method of improving hepatic sinusoidal blood flow, and that administration of αNOhRBC is an effective method not only for recovering the sinusoidal blood flow in the liver but also for correcting systemic acid-base equilibrium disorders (such as acidosis).

### EXAMPLE 4

In the Example, how adenosine contained in the extracellular fluid (outside of erythrocytes) effects ATP release from erythrocytes was examined in suspensions containing erythrocytes (RBCs). The method used was in accordance with Example 1 as follows.

Briefly, adenosine was added to the extracellular fluid of erythrocytes for 5 minutes. To a tightly sealed cuvette containing Krebs buffer adjusted to have a desired oxygen partial pressure within a range of 0-150 mmHg, the erythrocyte suspension was injected and the reaction was terminated 1 minute thereafter.

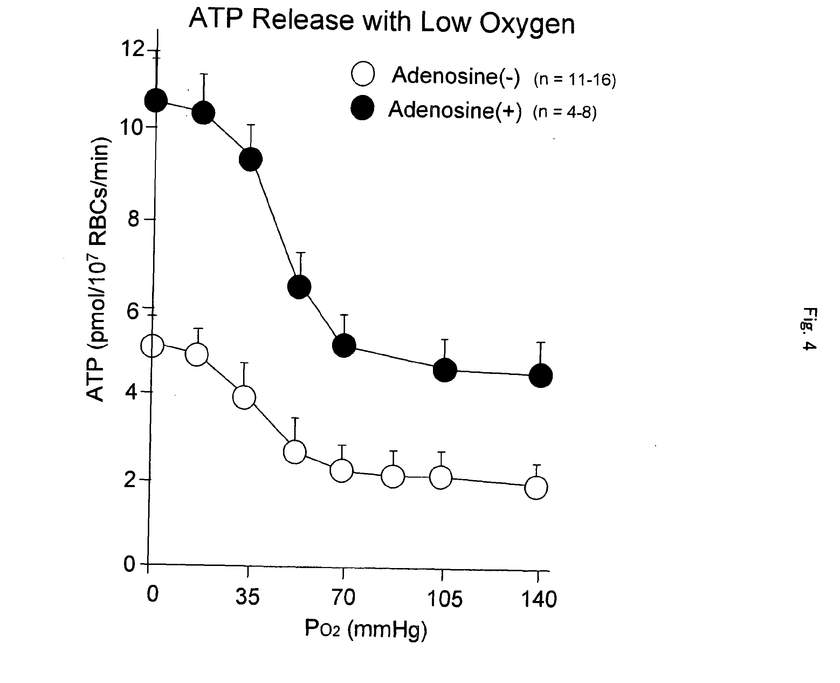

As a result, when 1 µmol/L adenosine was added to the extracellular fluid, both ATP release under normal oxygen concentration and ATP release under low oxygen for 1 minute were enhanced almost two times (Fig. 4).

Subsequently, the concentration-dependent effect of adenosine upon ATP release under low oxygen was examined.

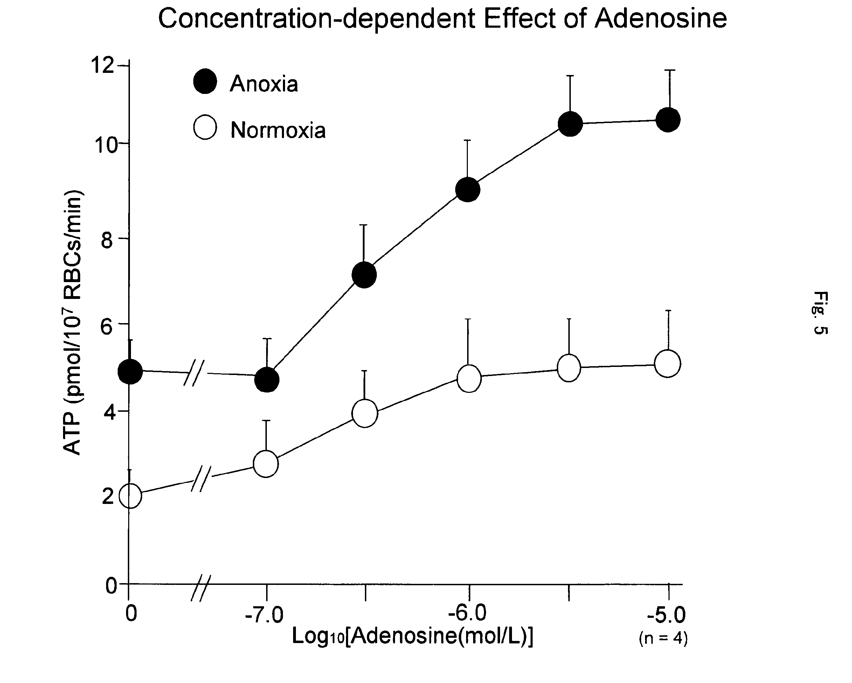

The results are shown in Fig. 5. In Fig. 5, "Anoxia" represents the results of measurement using Krebs buffer adjusted to have an oxygen partial pressure of 0 mmHg (no oxygen state: 1 minute treatment); and "Normoxia" represents the results of measurement using Krebs buffer adjusted to have an oxygen partial pressure of 100 mmHg (normal oxygen partial pressure in pulmonary alveoli and blood: 1 minute treatment). In Anoxia, it was shown that addition of only 1 µmol/L (10⁻⁶ mol/L) of adenosine increases ATP release about two times (**•** in Fig. 5).

From the results shown in Fig. 5, it is believed that addition of adenosine which serves as a raw material for ATP enhanced the amount of ATP. This means that addition of adenosine to blood conserved at a low temperature (e.g., blood for transfusion) can realize conservation of blood with high ATP release ability, suggesting that addition of adenosine is applicable to development of a new blood conservation method.

### EXAMPLE 5

It is an object of this Example to examine a membrane protein involved in ATP release. The method used was according to the method of Example 1.

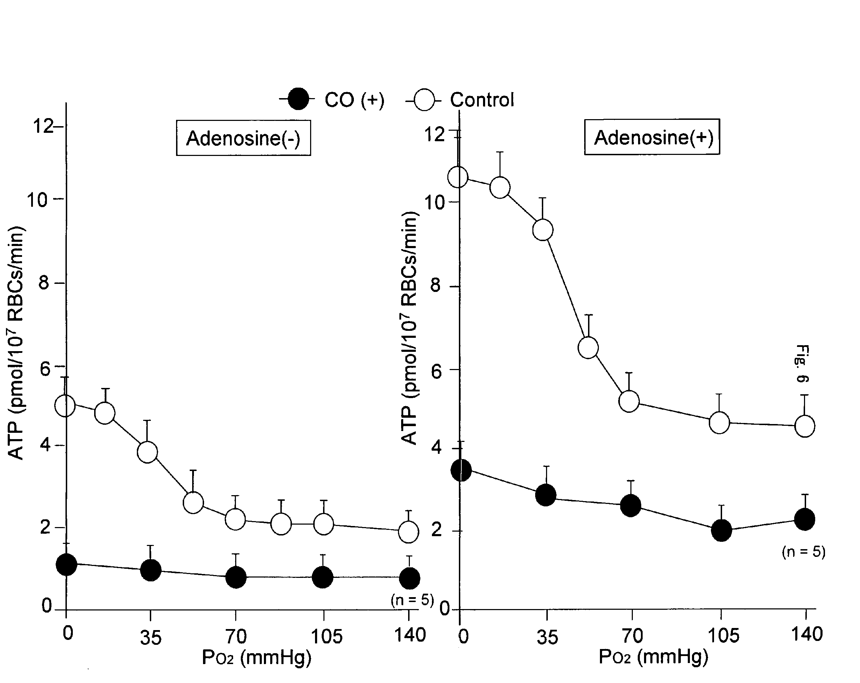

First, with carbon monoxide-treated erythrocytes (CO(+)), enhancement of ATP release under low oxygen was not confirmed. Even in the presence of 1 µmol/L adenosine, enhancement of ATP release due to low oxygen was extremely slight ("Adenosine(+)", • in Fig. 6). It was shown that when Hb is treated with CO to become the R-state, ATP release enhancement effect is eliminated or reduced. Mark "o" in Fig. 6 represents control.

Subsequently, the inventor examined from which site of the erythrocyte membrane ATP come out of cells. It is known that a membrane protein Band III (anion exchanger-1) exists in the erythrocyte membrane. As a drug which binds to the extracellular domain of Band III from the outside of cells and fills the hole of this channel protein, there is a sulfonylurea called DIDS (4,4'-diisothiocyanate-stilbene-2,2'-disulfonic acid) used for treating diabetes. Whether ATP is released via Band III in the erythrocyte membrane or not was examined using DIDS. First, the above examination was performed under conditions where ATP release enhancing effect was eliminated by CO treatment.

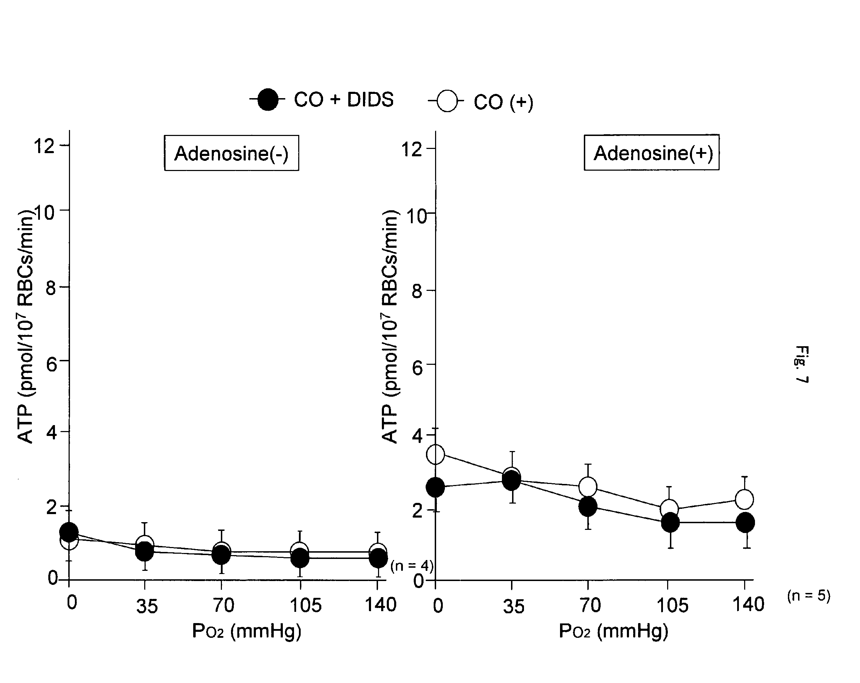

As a result, in the presence/absence of adenosine, there was no difference in the amount of ATP release between the case of DIDS addition to CO(+) erythrocytes and the case of no addition of DIDS (Fig. 7). From this result, the inventor could exclude the possibility that DIDS is involved in the enhancement ofATP release from erythrocytes in the R-state.

Subsequently, the concentration-dependent effect of DIDS was examined with CO-untreated erythrocytes.

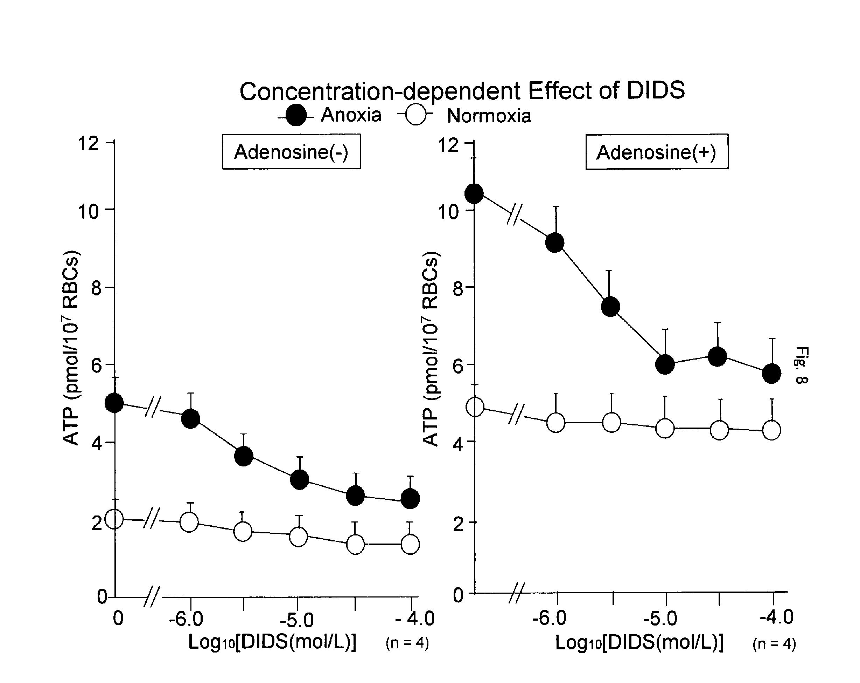

The results are shown in Fig. 8. In Fig. 8, "Anoxia" represents the results of measurement using Krebs buffer adjusted to have an oxygen partial pressure of 0 mmHg; and "Normoxia" represents the results of measurement using Krebs buffer adjusted to have normal oxygen partial pressure. The results revealed that ATP release increase due to low oxygen is inhibited depending on the concentration of DIDS (Fig. 8).

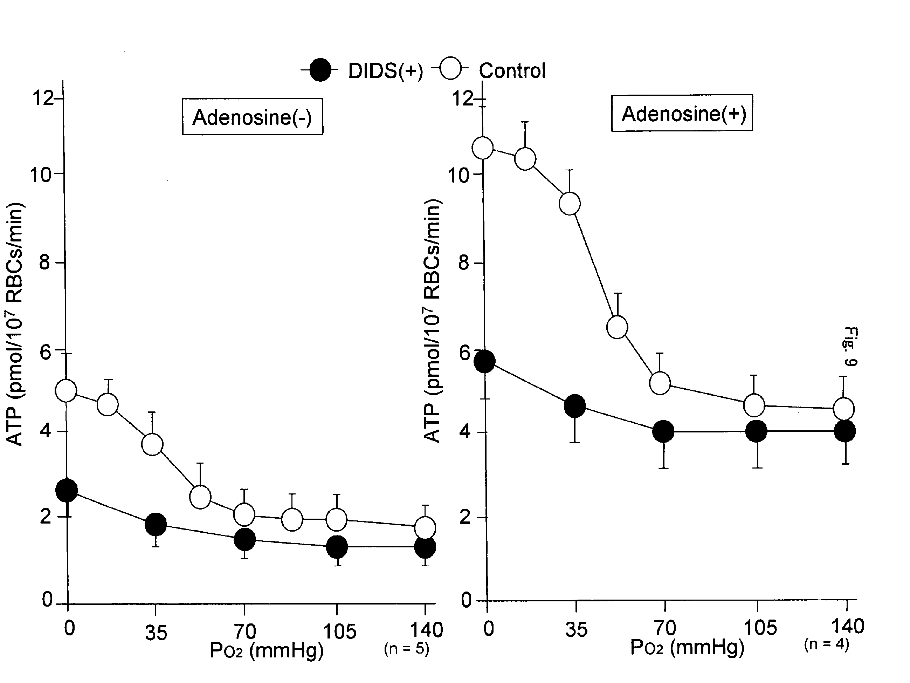

Further, the ATP release enhancement inhibitory effect of 10⁻⁴ mol/L DIDS was examined under oxygen partial pressures of 0-140 mmHg. The results revealed that DIDS inhibits ATP release increase due to low oxygen (Fig. 9).

Therefore, it was shown that ATP which was released in an increased amount under a no oxygen or low oxygen atmosphere is released extracellularly via Band III, an anion exchanger existing in the erythrocyte membrane.

It was also shown that the amount of ATP release from erythrocytes can be controlled freely by adding DIDS to the extracellular fluid.

### EXAMPLE 6

It is known that carbon dioxide has an effect called "hypercapnic protection". Briefly, when CO₂ concentration is increased by artificially reducing ventilation with mechanical respiration, the prognosis of ARDS (adult respiratory distress syndrome) is improved. In this Example, the hypercapnic protection effect of carbon dioxide was examined from the viewpoint of ATP release.

The method used was according to the method in Example 1. The amounts of ATP release when erythrocytes were exposed to various CO₂ partial pressures (P_{CO2}) from 0 to 80 mmHg for 5 minutes were measured.

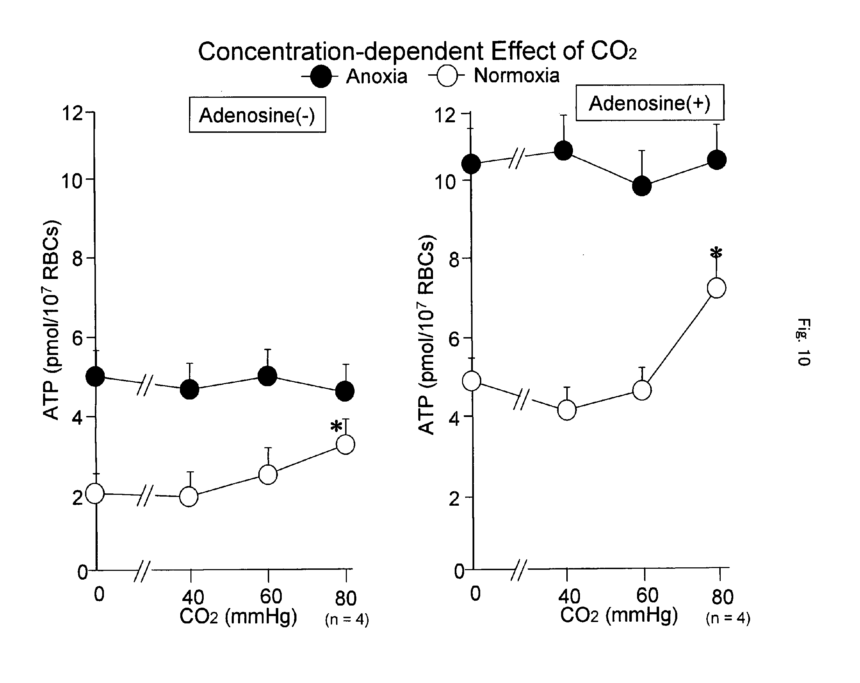

The results are shown in Fig. 10. In Anoxia (no oxygen state), ATP release was not affected by P_{CO2}. On the other hand, in normal oxygen state (Normoxia), ATP release was increased at 60-80 mmHg. At 80 mmHg, there was a significant difference regardless of adenosine treatment. In the presence of adenosine, a synergetic high effect was observed in ATP release enhancement. Therefore, according to the present invention, it was revealed that ATP release ability increases when the partial pressure of the carbon dioxide contained in the extracellular fluid of erythrocytes is increased under normal oxygen pressure.

### INDUSTRIAL APPLICABILITY

### EFFECT OF THE INVENTION

According to the present invention, a promoter for ATP release from erythrocytes, comprising a substance which stabilizes the structure of hemoglobin in erythrocytes in the T-state; and an inhibitor against ATP release from erythrocytes, comprising a substance which stabilizes the structure of hemoglobin in erythrocytes in the R-state are provided.

When the structure of hemoglobin is stabilized in the T-state, it is possible to allow ATP to be released from erythrocytes even under a high oxygen partial pressure. On the other hand, when the structure of hemoglobin is stabilized in the R-state, it is possible to inhibit ATP release from erythrocytes even under a low oxygen partial pressure. Therefore, the ATP release promoter of the invention is useful as a vasodilator or blood flow improver; and the ATP release inhibitor of the invention is useful as a vasoconstrictor, blood flow regulator, vasodilation inhibitor, or therapeutic for septic shock associated with hyperdilation of blood vessels.

Further, since erythrocytes in which the structure of hemoglobin is stabilized in the T-state are capable of releasing ATP and dilating blood vessels, the erythrocytes can enhance blood flow. On the other hand, erythrocytes in which the structure of hemoglobin is stabilized in the R-state are useful as a pharmaceutical preparation that can be administered to a pathology where peripheral blood vessels are suffering from hyperdilation because of septic shock or the like; and capable of preventing thrombi because these erythrocytes inhibit ATP release to thereby inhibit the activation of platelets.

## Claims

1. A promoter for ATP release from erythrocytes, comprising a substance which stabilizes the structure of hemoglobin in erythrocytes in the T-state.

2. The promoter according to claim 1, wherein the substance which stabilizes the structure of hemoglobin in erythrocytes in the T-state is at least one selected from the group consisting of bezafibrate, nitric oxide, carbon dioxide and adenosine.

3. The promoter according to claim 1 or 2, which is capable of releasing ATP extracellularly under an oxygen partial pressure of 100 mmHg or less.

4. A pharmaceutical composition comprising a substance which stabilizes the structure of hemoglobin in erythrocytes in the T-state.

5. The pharmaceutical composition according to claim 4, wherein the substance which stabilizes the structure of hemoglobin in erythrocytes in the T-state is at least one selected from the group consisting of bezafibrate, nitric oxide, carbon dioxide and adenosine.

6. The pharmaceutical composition according to claim 4 or 5, which is capable of releasing ATP extracellularly under an oxygen partial pressure of 100 mmHg or less.

7. The pharmaceutical composition according to any one of claims 4 to 6, which is a vasodilator or a blood flow improver.

8. A method of releasing ATP from erythrocytes, comprising stabilizing the structure of hemoglobin in the erythrocytes in the T-state.

9. The method according to claim 8, wherein ATP is released under an oxygen partial pressure of 100 mmHg or less.

10. An inhibitor against ATP release from erythrocytes, comprising a substance which stabilizes the structure of hemoglobin in erythrocytes in the R-state.

11. The inhibitor according to claim 10, wherein the substance which stabilizes the structure of hemoglobin in erythrocytes in the R-state is carbon monoxide or sulfonylurea.

12. The inhibitor according to claim 10 or 11, which is capable of inhibiting ATP release under an oxygen partial pressure of 100 mmHg or less.

13. A pharmaceutical composition comprising a substance which stabilizes the structure of hemoglobin in erythrocytes in the R-state.

14. The pharmaceutical composition according to claim 13, wherein the substance which stabilizes the structure of hemoglobin in erythrocytes in the R-state is carbon monoxide or sulfonylurea.

15. The pharmaceutical composition according to claim 13 or 14, which is capable of inhibiting ATP release under an oxygen partial pressure of 100 mmHg or less.

16. The pharmaceutical composition according to any one of claims 13 to 15, which is a vasoconstrictor or a blood flow regulator.

17. A method of inhibiting ATP release from erythrocytes, comprising stabilizing the structure of hemoglobin in the erythrocytes in the R-state.

18. The method of claim 17, wherein ATP release is inhibited under an oxygen partial pressure of 100 mmHg or less.

19. Erythrocytes in which the structure of hemoglobin is stabilized in the T-state.

20. The erythrocytes according to claim 19, which have been treated with at least one substance selected from the group consisting of bezafibrate, nitric oxide, carbon dioxide, adenosine and hydrogen ion.

21. The erythrocytes according to claim 19 or 20, which are capable of releasing ATP under an oxygen partial pressure of 100 mmHg or less.

22. Erythrocytes in which the structure of hemoglobin is stabilized in the R-state.

23. The erythrocytes according to claim 22, which have been treated with carbon monoxide or sulfonylurea.

24. The erythrocytes according to claim 22 or 23, which are capable of inhibiting ATP release under an oxygen partial pressure of 100 mmHg or less.

25. A pharmaceutical composition comprising the erythrocytes according to any one of claims 19 to 21.

26. The pharmaceutical composition according to claim 25, which is for treating ischemic diseases.

27. The pharmaceutical composition according to claim 26, wherein the ischemic disease is at least one selected from the group consisting of hemorrhagic shock, myocardial infarction, angina pectoris, cerebral infarction, intracerebral hemorrhage, obliterative arterial diseases, vascular disorders caused by diabetes, coronary artery stenosis, ischemic diseases of extremities, arteriosclerosis obliterans and ischemic ulcer/necrosis.

28. The pharmaceutical composition according to claim 26, wherein the ischemic disease is ischemia-reperfusion syndrome.

29. The pharmaceutical composition according to claim 28, wherein the ischemia-reperfusion syndrome results from at least one cause selected from the group consisting of post-shock resuscitation, perfusion after cold storage of organs, recanalization of blood flow after surgical operations, and reconstruction of obliterated blood vessels.

30. The pharmaceutical composition according to claim 25, which is for treating acidosis.

31. A pharmaceutical composition comprising the erythrocytes according to any one of claims 22 to 24.

32. The pharmaceutical composition according to claim 31, which is for treating vasodilatory diseases.

33. The pharmaceutical composition according to claim 32, wherein the vasodilatory disease is septic shock or anaphylactic shock.

34. A method of measuring ATP, comprising quantitatively determining the amount of ATP released from erythrocytes in an oxygen concentration-dependent manner.

35. A method of enhancing ATP release from erythrocytes, comprising adding adenosine to an erythrocyte suspension and exposing the resultant suspension to a no oxygen or low oxygen partial pressure condition.

36. The method according to claim 35, wherein the no oxygen or low oxygen partial pressure condition is a condition where oxygen partial pressure is 0-150 mmHg.

37. A method of enhancing ATP release from erythrocytes, comprising adding adenosine to an erythrocyte suspension and exposing the resultant suspension to a carbon dioxide partial pressure of 60-80 mmHg.

38. The method according to any one of claims 35 to 37, wherein the concentration of adenosine is 0.1-10 µmol/L.

39. A method of controlling ATP release from erythrocytes, comprising adding a substance that inhibits the anion permeation function of band 3 protein to an adenosine-added erythrocyte suspension.

40. The method of claim 39, wherein the substance that inhibits the anion permeation function of band 3 protein is sulfonylurea.

41. A controller for ATP release from erythrocytes, comprising a substance that inhibits the anion permeation function of band 3 protein.

42. The controller according to claim 41, wherein the substance that inhibits the anion permeation function of band 3 protein is sulfonylurea.

43. The controller according to claim 41 or 42, which is for inhibiting ATP release.
